# EUROPEAN PATENT APPLICATION

(11) **EP 1 688 151 A1**
(43) Date of publication of application: **09.08.2006**
(21) Application number: 06002395.9
(22) Date of filing: 02.02.2006
(51) Int. Cl.: A61L 9/20, A61L 9/22, F24F 3/00

(54) **Electro-optical air purifying and dust collecting device**

(30) Priority: 03.02.2005 HK 05100905
(71) Applicant: Yuen, Se Kit, Hong Kong (HK)
(72) Inventor: Yuen, Se Kit, Hong Kong (HK)
(74) Representative: Kerkhoff, Thomas

(57) **Abstract**

This invention relates to an air purifier that uses both transportation vehicles battery and conventional indoor power supply, employs ultraviolet radioactive rays to kill bacteria, viruses and molds in the air and to increase amount of anions. This air purifier has an infrared remove controller and a ability of providing illuminations. This air purifier draws indoor air in and out of the air purifier through spiral air pipe, which contains many rifle spiral shape air collectors, kills bacteria, viruses and moulds in the air and discharges electricity by means of negative high voltage output through carbon fibers to produce anions, so as to improve quality of the air.

## Description

### Background of the Present Invention

### Field of the Present Invention

The present invention is related to an air purifier and more particularly it relates to a electro-optical air purifier with a dust collector.

### Description of Related Arts

In this modem society, illuminating apparatus is a must for families, hospitals, senior citizen centers, shopping malls, theaters, restaurants, office buildings, automobile manufacturers, elevators, various cars, ships, airplanes and trains. Meanwhile, air purifies are commonly used in nowadays. Especially problems of air pollution are getting worst as our society has become more industrialize, more depending on transportation vehicles, and more diseases are transmitted by air.

Most conventional air purifiers require normal indoor power supply such as 220V or 120 volt depending on geographical limitation, while few of those are designed mainly for transportation vehicles, which allow air purifier to function under a power supply generated by automobile, i.e. 12 volt, as transportation vehicles are operating. Almost no conventional air purifier has an ability providing capacity for both indoor power supply and automobile power supply.

Moreover, most conventional air purifiers are designed to function as an independent unit without any ability of illumination. A consumer must purchase an additional illuminating equipment and install it next to an air purifier should he/she wants to have lighting available while enjoying a purified air. In additions, most conventional air purifiers are designed without any infrared ability which limit consumer an ability to turn on and off freely or from a distance while operating such units.

Worsen still; most conventional air purifiers can only separate unwanted airborne particles from air to achieve so-called purification for air. However, this is a well known fact that anions in the air are good for health. It would be beneficial should air purifiers can produce anions while separating unwanted airborne particles from air allows.

### Summary of the Present Invention

A main object of the present invention is to provide an air purifier has an ability providing capacity for both indoor power supply and automobile power supply.

Another object of the present invention is to provide an air purifier has ability of illumination while purifying the air simultaneously or respectively.

Another object of the present invention is to provide an air purifier has infrared capacity which allow user to control a air purifier within a distance without physically touching it.

Another object of the present invention is to provide an air purifier can produce anions while separating unwanted airborne particles from air flows.

Accordingly, in order to accomplish above objects, the present invention provides an air purifier, which comprises:
a three-dimensional rectangular housing
a spiral air pipe,
an ultraviolet emission tube with pre-determined size is placed inside of said
spiral air pipe, and
a ventilator, ultraviolet emission tube, negative high voltage electrical
carbonization fiber, remote controller and fluorescent lamp tube.
Said three-dimensional rectangular housing further comprises of:
incoming and outgoing air vents, said negative high voltage carbonization fiber is fixed inside of said outgoing air vent, ultraviolet tube is located at a front portion of said air incoming vent, and said ventilator is placed in a center portion of said three-dimensional rectangular housing.

Said air purifier intakes air that contains viruses, bacteria, and mildews into said air purifier through said incoming air vent. There is a rifle 1 like spiral strip inside of said air purifier to direct moving air passing through Photoelectric air sterilization lattice into said outgoing air lattice. Forcing viruses, bacteria and mildews in the air to contact ultraviolet emission tube, so as said ultraviolet could perish viruses, bacteria and mildews contained in the air effectively. Sanitized air then go through said ionizer, which generates negative ions to increase its ratio in the air thus released fresh air through said air purifier's air outlet will be able to improve indoor air quality.

Other than on and off switch that connects with a power source, the present invention also provide with a remote controller. It allows user to remotely control all of functions and features such as turning on, turning off, to increase or decrease power or level of air out and/or input, to increase and/or decrease level of ions, and/or turning on and off illumination.

The present invention provides an environmental electro optical extreme ultraviolet radiant sterilizer that kills germs in the air with ultraviolet ray on one side, and generates anions to benefit physical and mental health of man as well as static electric dust-absorbing function on the other side.

In order to serve said purposes, this new practical invention provides an environmental electro optical extreme ultraviolet radiant sterilizer composed of a base, including a coat with openings at both top and bottom with the bottom end connected to the chassis in a movable manner. There are air grids at all peripheral sides of the said coat, dust filter mesh at inside, an anion drive circuit and a positive electromagnetic-field drive circuit on the chassis. There is a body above the said base and an outside coat, which consists of front and rear portions. The front coat installs operational control panel, and the rear coat installs slide-in air grid, through which the coat is fastened to the chassis at the coat opening on the top. Inside the coat there is an air collector that contains an air chamber connected to a flare exhaust outlet on the top and an intake entry at the bottom. The inner surface of the chamber contains a convex-concave device that buffers air in flowing upward and titanium oxide catalyst, and at least one ultraviolet sterilizing tube. The said flare exhaust outlet has an air extractor on the top. Between the intake entry and the flare exhaust outlet of the extractor there is a high-pressure discharging carbon fiber cable that generates anions after being connected to the said anion drive circuit output power. The flare inlet under the air chamber is sealed up with the opening on the top of the base, enabling the extractor to draw air into the collector, and release air from the top after sterilization treatment with ultraviolet sterilizing radiant ray. There are at least one positive electromagnetic-field durst collectors on the surface of the said coat.

There are two positive electromagnetic-field dust collectors.

Said positive electromagnetic-field dust collector consists of a conductive sponge and a bracing strut, which is joined with the outer surface of the coat in a movable manner. A high-voltage copper sheet is wrapped between the conductive sponge and the bracing strut. The high-voltage copper sheet is connected to the said positive electromagnetic-field drive circuit to cause the conductive sponge to carry positive ions.

There is also an upright tube exchange stand, which consists of a slide-in check board and a handle bar for the installation of the said ultraviolet sterilizing tube. Said cylinder air chamber has a vertical opening on the top slot, with which the slide-in check board is fastened to seal up the air within the chamber. The said rear coat also has an opening that is connected to the handle bar in a movable manner.

Said ultraviolet sterilizing tube's radiant wave is 250nm-380nm long.

Said ultraviolet sterilizing tube's radiant wave length is 253.7nm.

There is also a flare exhausting grid at the outlet of the intake fan that enables release of air into the peripheral environment.

As this new practical environmental electro optical extreme ultraviolet radiant sterilizer conducts initial filter of relatively large particles in the air, and contains titanium oxide catalyst air collector, carbon fiber cable anion-generating device, and positive electromagnetic dust collector, it can easily absorb negative charge particles while preventing black precipitation pollution. The collection neither resembles air filter nor depends on movement of indoor air, but on capture of particles carrying negative charges by conductive sponge that carries positive charges. The ability to capture is determined by the strength of the electrostatic force of positive electrostatic field generated by the conductive sponge. Not only can positive electromagnetic duct collector generate electromagnetic field at 10Hz frequency to benefit human health, it can also capture and eliminate h armful pollutants with diameters 1 ess than 0.01mm a float in the indoor air, such as allergy-causing particles, pollen, dust, smog, powder and odorous particles and to refresh the air. It can also capture particles carrying negative electric charges generated by the anion air purifier to eliminate black precipitating pollution. In addition, increasing the amount of anions in the indoor air can enhance biochemical actions (the amount of anions inhaled) and reduce secretion of hormones that cause depression and fatigue. The extreme ultraviolet radiant ray and Naomi Ti02 optic catalyst can effectively eliminate bacteria, viruses and moulds in the air, as well as poison organics.

### Brief Description of the Drawings

Figure 1 is an assembly view of the present invention.
Figure 2 is a sectional view thereof.
Figure 3 is a detail view of filter thereof.
Figure 4 is a rear view of thereof.
Figure 5 is a rear view with filter door opened thereof.
Figure 6 is a section A-A view thereof.
Figure 7 is a section B-B view thereof.
Figure 8 is front isometric view thereof.
Figure 9 is a front isometric view with filter door opened thereof.

### Detailed Description of the Preferred Embodiment

Detailed illustrative description of implementation of the new practical invention is hereunder given in combination with attached pictures.

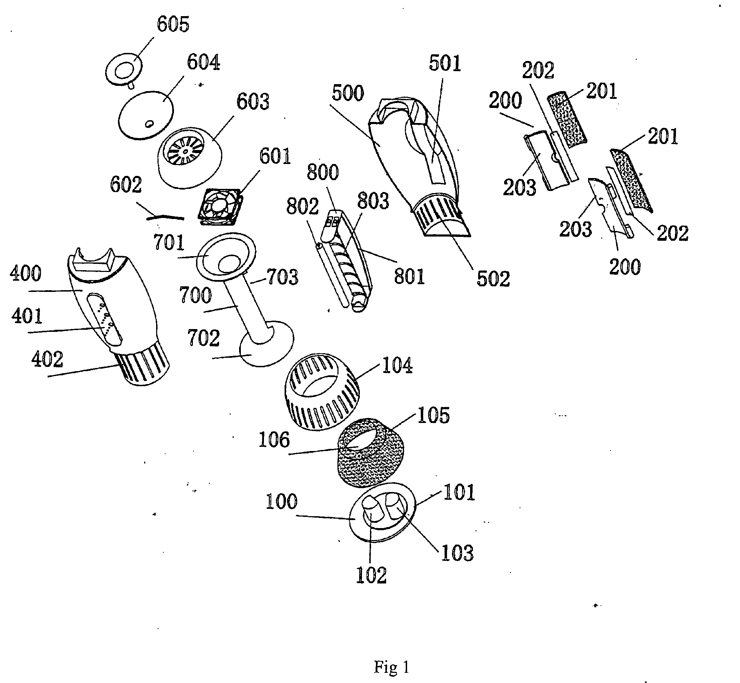

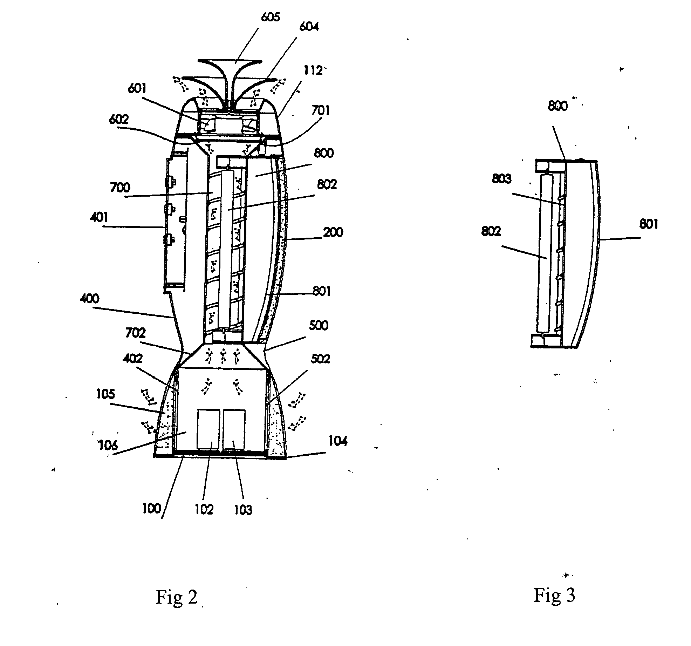

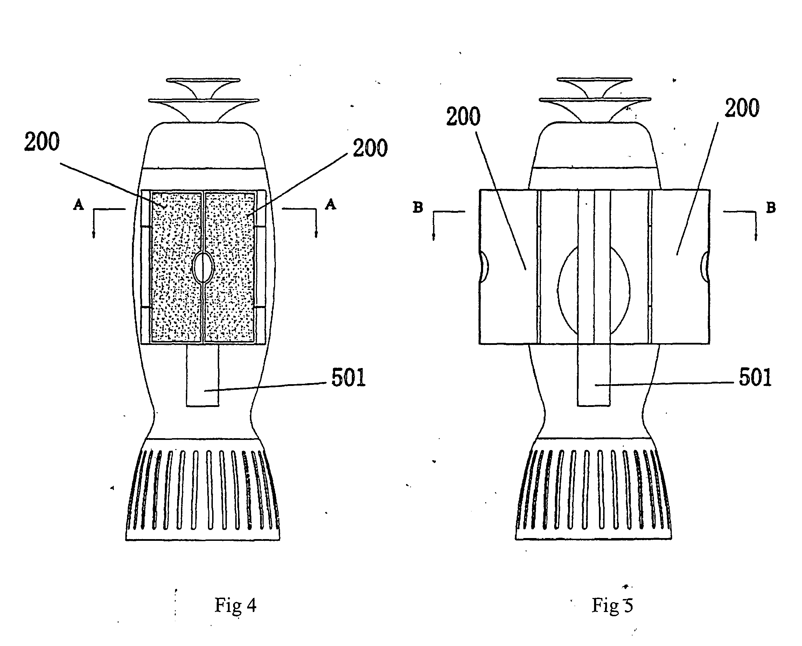

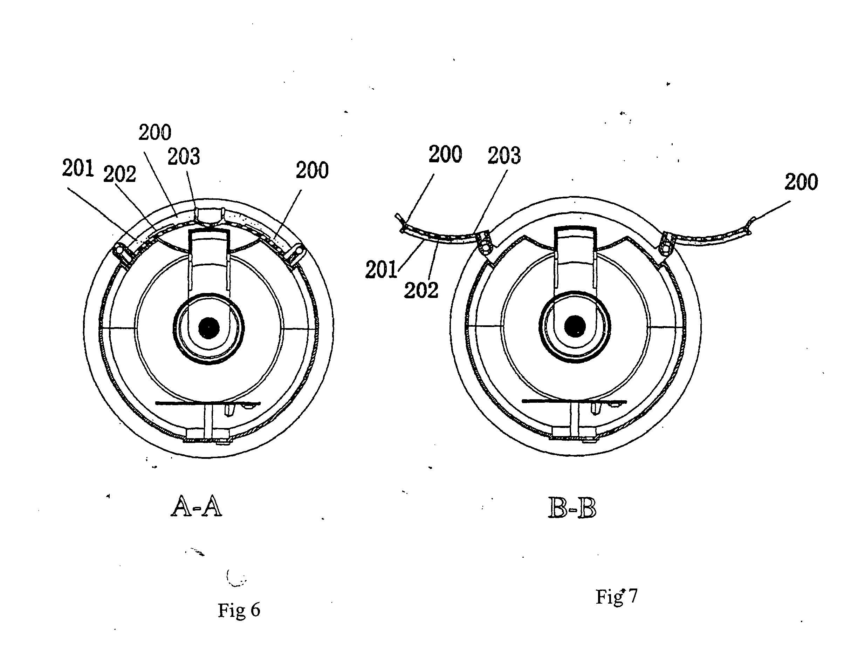

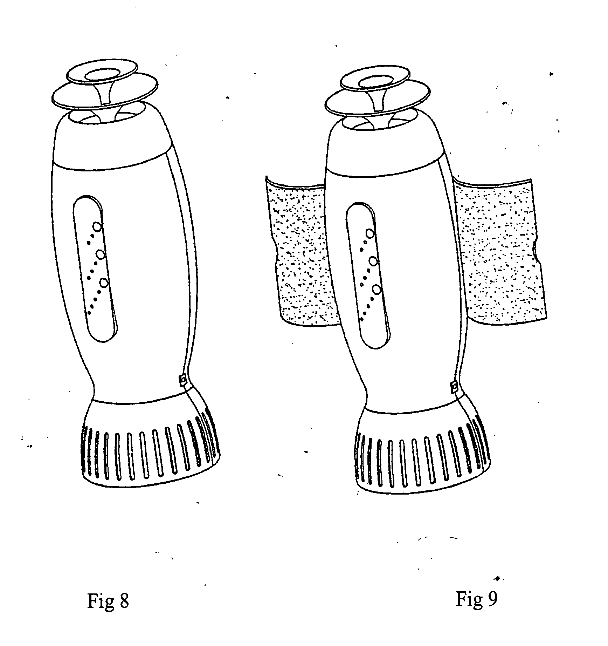

This environmental electro optical extreme ultraviolet radiant sterilizer as described in Fig. 1 and Fig. 2 consists of a base 100, which is composed of a chassis 101, an anion drive circuit 102 and a positive electromagnetic-field drive circuit 103 in the chassis, a coat 104 with opening on both top and bottom ends and connected to the chassis 101 in movable manner, air filter grid on all sides of the coat 104, dust filter mesh 105 in the coat that filters air entering the base 100, a body above the base 100 that includes a coat and an air collector. The coat is divided into front coat 400 and rear coat 500. The front c oat 4 00 contains an operational control panel 401 in the surface and slide-in air grids 402 at the bottom. The rear coat 500 contains an opening 501 in the center and a slide-in air grid 502 at the bottom. After combining the front and the rear coats, two slide-in air grids at each bottom run through the opening on the top of the coat 104 to reach the chassis 101 so as to secure the body and to form an air filter chamber 106 within the base 100. The air collector consists of a vertical air chamber 700 with a concave 703, and an upright tube exchange stand 800 for installation of ultraviolet sterilizing tube at 253.7nm wavelength. The upright tube exchange stand 800 as described in Fig. 3 includes an arc handle bar 801, ultraviolet sterilizing tube 802 and base standing check board 803. The check board 803 is secured within the concave 703 to seal up the air chamber 700. The arc handle bar 801 is connected to the opening notch 501 on the coat 500 in a movable manner so that the upright tube exchange stand 800 may be drawn out of the coat for the convenience of exchange of ultraviolet sterilizing tube 802. The cylinder air chamber 700 is respectively connected to a flare exhaust outlet 701 and an air entry 702 at top and bottom. The flare exhaust outlet has an intake fan 601 on the top, an upper coat 603 of air vent on the outer surface of the intake fan 601, on the intake entry of which there is high-voltage discharging carbon fiber cable 602. The carbon fiber cable is connected to the outlet of the anion drive circuit 102 to generate anions, while the flare air entry 702 at the bottom of the air collector is connected to the upper opening of the said stand. In order to extend the length of retention within the cylinder air chamber 700 so as to achieve better sterilizing results with ultraviolet ray, the air chamber 700 installs an upward spiral hump or buffering device against up-flowing air in other shapes on the inner surface. A upward spiral notch in the inner surface of the chamber, for instance, can help effectively kill bacteria and germs in the air flowing upward. The titanium oxide catalyst paint on the inner surface of the cylinder air chamber 700 helps decompose harmful organic air such as methanol and benzene. In the outer surface of the rear coat 500 there are a pair of positive electromagnetic dust collectors 200, which could be practically installed in any part of the coat surface in the amount of one or more. The said positive electromagnetic dust collector 200 consists of one conductive sponge 201, one high-voltage copper sheet 202 and one bracing strut 203. The bracing strut 203 is closely attached to the outer surface of the rear coat 500 by hinge connection or.other flexible connection for the convenience of usage and storage. Fig. 5, 7 and 9 demonstrated the positive electromagnetic dust collector in its unfolding status on the surface of the coat, and Fig. 4, 6 and 8 demonstrated the positive electromagnetic dust collector in its folding status on the surface of the coat. The high-voltage copper sheet 202 is secured to the bracing strut 203, and the conductive sponge 201 is wrapped around the high-voltage copper sheet 202, which is connected to the output end of the positive electromagnetic drive circuit 103 to enable the conductive sponge 201 to carry positive ions. The flare exhaust grid 604 at the vent of coat 603 enables the fresh air to emit into the environment after proper treatment. There is also a handle bar 605 on the top of the exhaust grid 604 that can be folded up to store or carry.

Working principles of this environmental electro optical extreme ultraviolet radiant sterilizer are as follows: the indoor air runs in through the grid in the coat 104, and is filtered through the dust filter mesh 105 to eliminate relatively large particles of dust, then enters into the air filter chamber 106, where the air is drawn into the air collector via the intake fan 601 to reach the cylinder air chamber 700. The air further goes through physical radiation by 253.7nm length of ultraviolet sterilizing wave in the sterilizing tube 802 and chemical treatment of titanium oxide at inner surface, and then exits through the flare exhaust outlet 701 at the top. Meanwhile, the carbon fiber cable 602 generates high-voltage power to create large amount of anions beneficiary to physical and mental health of man. Most of these anions attach onto the tiny particles in the air. As the conductive sponge 201 of the positive electromagnetic-field dust collector 200 in the coat carries positive ions, particle dusts carrying anions can be drawn to attach the conductive sponge 201, so as to avoid black precipitation pollution in the indoor air. On the other hand, the positive electromagnetic-field duct collector 200 can generate electromagnetic-field at 10Hz frequency to benefit human health.

## Claims

1. An electro optical air purifying & dust collector comprises:
a base, including a coat with opening on both top and bottom, and the bottom opening is joined with chassis in movable manner; said coat has inlet grid in the outer surface and
dust filter mesh in the inner surface; said chassis has an anion drive circuit and a positive electromagnetic drive circuit; and
a body sitting on the said base, and
an outside coat, which is divided into front coat and rear coat, while the front coat contains operational control panel, and the bottom coat contains a slide-in grid, through which said coat is inserted and fastened to the chassis from the opening on the top of the coat; and
there are air collectors in the coat that consist of a chamber with a flare exhaust outlet and
an air entry at top and bottom; and
an inner surface of the air chamber contains convex-concave device that buffers air from moving upward and titanium catalyst agent, and at least one ultraviolet sterilizing tube;
said flare exhaust outlet has an extraction device at the top; and
between the intake port of the extraction device and the flare exhaust outlet, there is a high voltage discharge carbon fiber cable, which connects to the output port of the anion drive circuit to generate anions;
said flare intake entry under the air chamber is connected to the opening on the top coat, so that the air within the base is drawn upward into the air collector, where it goes through ultraviolet sterilizing tube radiation treatment before running out from above;
said coat has at one positive electromagnetic-field dust collector on its surface.

2. An electro optical air purifying & dust collector as recited in claim 1, said electro optical is further comprises two positive electromagnetic-field dust collectors.

3. An electro optical air purifying & dust collector as recited in claim 1, said dust collector is further comprises one conductive sponge and one bracing strut, which is connected to the outer surface of the s aid c oat, a high-voltage copper sheet that is wrapped between a conductive sponge and the bracing strut, and connected to the output power of said positive electromagnetic- field drive circuit so as to cause the conductive sponge to carry positive ions.

4. An electro optical air purifying & dust collector as recited in claim 1, is featured by an upright tube exchange stand, which consists of a standing check board and a handle bar for installation of the said ultraviolet sterilizing tube. The said cylinder air chamber has a vertical notch on the top, which secures the standing check board to seal up the air within the chamber. The said coat also has an opening that connects to the handle bar in a flexible manner.

5. An electro optical air purifying & dust collector as recited i n claim 1, s aid electro optical release a wavelength of the said ultraviolet sterilizing tube is 250nm - 380nm.

6. An air purifying & dust collector, which comprises:
a three-dimensional rectangular housing
a spiral air pipe,
an ultraviolet emission tube with pre-determined size is placed inside of said
spiral air pipe, and
a ventilator,
an ultraviolet emission tube,
an negative high voltage electrical carbonization fiber,
a remote controller and
a fluorescent lamp tube.
Said three-dimensional rectangular housing further comprises of:
incoming and outgoing air vents, said negative high voltage carbonization fiber is fixed inside of said outgoing air vent, ultraviolet tube is located at a front portion of s aid air incoming vent, and said ventilator is placed in a center portion of said three-dimensional rectangular housing.
There is a rifle like spiral strip inside of said spiral air pipe inside said three-dimensional rectangular housing to direct moving air passing through said air sterilization lattice into said outgoing air lattice.

7. An electro optical air purifying & dust collector as recited in claim 6, said three-dimensional rectangular housing is having a spiral air pipe;
said spiral air pipe further comprises of:
a rifle like spiral strip, which allows direct more air passing through said air sterilization lattice into said outgoing air lattice located at both ends of said body.

8. An air purifying & dust collector comprises:
a three dimensional housing, and
at least one set of circuit board, and
at least one anion-generating circuit,
at least one switch,
at least one ultraviolet radioactive ray chamber, and
one air filter frame grid made of rubber or metal material and shaped in rectangular structure,
at least on infrared remote controller and
at least one power input line; and
said three-dimensional rectangular housing further comprises of:
incoming and outgoing air vents, said negative high voltage carbonization fiber is fixed inside of said outgoing air vent, ultraviolet tube is located at a front portion of s aid air incoming vent, and said ventilator is placed in a center portion of said three-dimensional rectangular housing.

9. As recited in claim 1, said air purifier is equipped with an electrical circuitry system and a negative ion generator to increase negative ions and purify air in an indoor environment. Said negative ion generation circuitry, and activate circuitry for ultraviolet emission tube that produces ultraviolet wavelength of 253.7 nanometers.

10. As recited in claim 6, said air purifier is equipped with an electrical circuitry system and a negative ion generator to increase negative ions and purify air in an indoor environment. Said negative ion generation circuitry, and activate circuitry for ultraviolet emission tube that produces ultraviolet wavelength of 253.7 nanometers.

11. As recited in claim 1, said air purifying & dust collector is equipped with an electrical circuitry system and a negative ion generator to increase negative ions and purify air in an indoor environment. Said negative ion generation circuitry, and activate circuitry for ultraviolet e mission tube that produces ultraviolet wavelength of 253.7 nanometers and said output value of negative high voltage should be between 4,500 to 8,500 voltages. Said function of the activation circuitry's changeable impedance on ultraviolet tube, which generates ultraviolet at 253.7nm, is to adjust current's output value.

12. As recited in claim 6, said air purifying & dust collector is equipped with an electrical circuitry system and a negative ion generator to increase negative ions and purify air in an indoor environment. Said negative ion generation circuitry, and activate circuitry for ultraviolet emission tube t hat produces ultraviolet wavelength of 253.7 nanometers and said output value of negative high voltage should be between 4,500 to 8,500 voltages. Said function of the activation circuitry's changeable impedance on ultraviolet tube, which generates ultraviolet at 253.7nm, is to adjust current's output value.
